# EUROPÄISCHE PATENTSCHRIFT

(11) **EP 2 048 974 B1**
(45) Veröffentlichungstag und Bekanntmachung des Hinweises auf die Patenterteilung: **10.12.2014**
(21) Anmeldenummer: 07785950.2
(22) Anmeldetag: 09.07.2007
(51) Int. Cl.: A23L 1/307, A23L 1/30, A23L 1/236, A23L 1/29, A23L 2/60, A23L 2/385, A23G 1/56, A23G 3/42, A21D 2/18, A23L 1/09, A23G 1/40, A23C 9/152

(54) **VERWENDUNG VON ISOMALTULOSE IN REGENERATIV WIRKENDEN NAHRUNGSMITTELN**
USE OF ISOMALTULOSE IN FOOD PRODUCTS HAVING A REGENERATIVE EFFECT
UTILISATION D'ISOMALTULOSE DANS DES ALIMENTS AYANT UNE ACTION REGENERATIVE

(30) Priorität: 31.07.2006 DE 102006035912
(43) Veröffentlichungstag der Anmeldung: 22.04.2009
(73) Patentinhaber: Südzucker Aktiengesellschaft Mannheim/Ochsenfurt, 68165 Mannheim (DE)
(72) Erfinder: BERG, Aloys, 79183 Waldkirch (DE); KÖNIG, Daniel, 79104 Freiburg (DE); KOWALCZYK, Jörg, 67304 Eisenberg/Steinborn (DE); KOZIANOWSKI, Gunhild, 67269 Grünstadt (DE); THEIS, Stephan, 67146 Deidesheim (DE)
(74) Vertreter: Schrell, Andreas
(86) Internationale Anmeldenummer: PCT/EP2007/006079
(87) Internationale Veröffentlichungsnummer: WO 2008/014871

(56) Entgegenhaltungen:
- WO-A-2005/013720
- JP-A- 2000 300 212
- DATABASE WPI Week 200611 Derwent Publications Ltd., London, GB; AN 2006-105150 XP002462966 & JP 2006 022068 A (MEIJI MILK PROD CO LTD) 26. Januar 2006 (2006-01-26)
- DATABASE WPI Week 198915 Derwent Publications Ltd., London, GB; AN 1989-112222 XP002462967 & JP 01 060360 A (MITSUI SEITO KK) 7. März 1989 (1989-03-07)
- DATABASE FSTA [Online] INTERNATIONAL FOOD INFORMATION SERVICE (IFIS), FRANkFURT-MAIN, DE; ARVANITI K ET AL: "Reproducibility of energy and macronutrient intake and related substrate oxidation rates in a buffet-type meal." XP002462965 Database accession no. 2000-00-a1384 & ARVANITI K ET AL: BRITISH JOURNAL OF NUTRITION, 2000, BRITISH JOURNAL OF NUTRITION 83 (5) 489-495 2000 CORRESPONDENCE (REPRINT) ADDRESS, A. TREMBLAY, CENT. FOR RES. ON ENERGY METABOLISM, LAVAL UNIV., QUE., CANADA. FAX +1 418 656 2441. E-MAIL ANGELO.TREMBLAY(A)KIN.MSP.ULAVAL.CA
- LANDRY NANCY ET AL: "Whole-body fat oxidation rate and plasma triacylglycerol concentrations in men consuming an ad libitum high-carbohydrate or low-carbohydrate diet." AMERICAN JOURNAL OF CLINICAL NUTRITION, Bd. 77, Nr. 3, März 2003 (2003-03), Seiten 580-586, XP002462964 ISSN: 0002-9165

## Beschreibung

Die vorliegende Erfindung betrifft neue Verwendungen von Isomaltulose und diese enthaltenden Gemischen.

Nach gegenwärtigen Ernährungsempfehlungen sollen mindestens 50 % der täglichen Energiezufuhr in Form von Kohlenhydraten bereitgestellt werden. Dabei werden insbesondere niedrig-glykämische Kohlenhydrate empfohlen, da hierdurch hohe Blutglucose- und Insulinreaktionen vermieden werden können. Mit Kohlenhydraten und speziell Glukose können Muskeln, Gehirn und Nerven gleichermaßen gut arbeiten. Erythrozyten des Blutes, das Nierenmark und das Nervensystem sind obligat auf Glukose als Energiequelle angewiesen. Ein Kohlenhydratmangel im Stoffwechsel aufgrund einer zu geringen Kohlenhydrataufnahme führt zu Hypoglykämie, verminderter Glukosetoleranz, Ketose und Störungen im Bereich des Wasser- und Mineralstoffhaushaltes. Eine Aufrechterhaltung des Blutglukose-Spiegels ist vor allem für die kontinuierliche Versorgung der Gehirn- und Nervenzellen sowie der Blutzellen mit Glukose als Energiequelle wichtig. Von Nachteil ist die relativ begrenzte Speicherfähigkeit von Kohlenhydraten im Organismus. Die Speicherform der Kohlenhydrate ist das Glykogen in der Leber und Muskulatur.

Auch bei länger dauernden körperlichen oder sportlichen Einsätzen kommt es zur Entleerung der Glykogenspeicher. Je größer die Belastungsintensität ist, desto größer ist der Kohlenhydratanteil an der Energiebereitstellung und desto mehr werden die Glykogenspeicher entleert. Dies führt zur Störung der Blutglukosehomöostase, was auch die Konzentrations- und Koordinationsfähigkeit beeinträchtigt und zur Erschöpfung führt. Es gilt daher als vorrangiges Ernährungsziel, durch Kohlenhydrat-reiche Kost für optimale Glykogenspeicher zu sorgen und durch Verzehr von ausreichend Kohlenhydraten auch während körperlicher Tätigkeit soviel Kohlenhydrat wie möglich und auch in länger dauernden Aktivitäten und sportlichen Einsätzen als Energiequelle zur Verfügung zu haben.

In diesem Zusammenhang ist eine hohe Fettoxidation zur Energiebereitstellung bei körperlichen Aktivitäten vorteilhaft, da sie die Glykogenreserven, z.B. auch für spätere Belastungsphasen oder den Schlussspurt, schont. Es ist allgemein bekannt, dass durch Training die Fettoxidation bei einer bestimmten Belastungsintensität erhöht werden kann. Je besser ein Individuum trainiert ist, desto höher ist die Fettoxidation. Das Ziel von körperlichem Training ist daher unter anderem insbesondere die Optimierung und Anpassung der Fettverbrennung. Ein Trainingserfolg zeigt sich demnach auch in Form einer erhöhten Fettoxidation.

Eine hohe Fettverbrennung ist weiterhin vorteilhaft zur Gewichtskontrolle und hinsichtlich der Prävention und Therapie von Übergewicht und Folge- oder Begleiterkrankungen wie Diabetes, gestörter Glukosetoleranz, Fettstoffwechselstörungen, Arteriosklerose, dem metabolischen Syndrom, Lebererkrankungen und anderen Stoffwechselerkrankungen. So ist bekannt, dass eine niedrige Fettverbrennung zu einer positiven Fettbilanz und einem metabolischem Energieüberschuss führt und die Entstehung von Übergewicht bewirkt bzw. der Gewichtskontrolle und dem Abnehmen entgegenwirkt. Strategien zur Prävention und Behandlung von Übergewicht und Folgeerkrankungen streben daher eine Steigerung der Fettverbrennung und Fettoxidation und Erzielung einer negativen Fett- und Energiebilanz an. Andererseits ist jedoch bekannt, dass ein Verzehr von Kohlenhydraten die Fettoxidation herabsetzt, die Kohlenhydrat-Oxidation fördert und zu einem höheren respiratorischen Quotienten führt. Der respiratorische Quotient (RQ) spiegelt das Verhältnis von CO₂/O₂ in der Atemluft wieder und ist ein Maß dafür, welche Nährstoffe verbrannt werden. Reine Kohlenhydratverbrennung führt zu einem respiratorischen Quotient von 1, während reine Fettverbrennung zu einem respiratorischen Quotienten von 0,7 führt. Diese nachteilige Wirkung, d.h. die Einschränkung der Fettoxidation, hält über viele Stunden nach Verzehr der Kohlenhydrate an. Ferner ist bekannt, dass die Fettoxidation auch dann noch anhaltend reduziert ist, wenn man sich sportlich bzw. körperlich betätigt (Achten & Jeukendrup, 2003). So war bei einem Verzehr von Kohlenhydraten vor körperlicher Betätigung die gesamte Fettoxidation über eine 8h-Periode um ca. 30 % herabgesetzt (Schneiter et al., 1995). Des Weiteren ist bekannt, dass bei einem Kohlenhydrat-Verzehr während sportlich-körperlicher Betätigung freie Fettsäuren (FFS) schneller abfallen, als wenn keine Kohlenhydrate verzehrt werden. Die Fettoxidation wird somit durch Kohlenhydrate beeinträchtigt, da schneller eine Umstellung von Fettoxidation auf Fett-Speicherung erfolgt. Diese Reduktion der Lipolyse und Fettoxidation nach Kohlenhydrat-Verzehr, die auch über das Belastungsende hinaus anhält, trägt zu einem Energieüberschuss und zur Förderung der Fetteinlagerung bei.

Im Vergleich von Kohlenhydratquellen mit unterschiedlicher Wirkung auf den Blutglukose- und Insulinspiegel gelten häufig niedriger glykämische Kohlenhydrate als vorteilhaft. Diese würden zu einer geringeren Reduktion der Fettoxidation führen als höher glykämische Kohlenhydrate. Andererseits ist jedoch bekannt, dass es sowohl nach dem Verzehr von Lebensmitteln mit hohem GI (glykämischer Index) als auch von Lebensmitteln mit niedrigem GI zur Reduktion der Fettoxidation kommt und dass die Fettoxidation niedriger ist, als wenn überhaupt keine Kohlenhydrate verzehrt werden (Brand-Miller et al., 2002). Auch ist bekannt, dass Fructose, ein Kohlenhydrat mit niedrigem GI und geringer Insulin-Wirkung sogar eine größere Reduktion der Fettoxidation bewirkt als Glukose (Tittelbach et al., 2000). Ein Verzehr von Kohlenhydraten führt somit allgemein zu einer geringeren Fettoxidation.

Es ist schließlich bekannt, dass Änderungen, die sich im respiratorischen Quotienten eines Individuums während körperlicher Betätigung ergeben, nach Belastungsende kompensiert werden. So zeigte sich bei einem höheren respiratorischen Quotienten während hochintensiver Belastung ein niedrigerer respiratorischer Quotient in der Nachbelastungsphase, während der respiratorische Quotient während niedrig-intensiver Belastung geringer und nach Belastungsende höher war (Saris et al., 2004). Dionne et al. (1999) fanden, dass es bei Verzehr eines Kohlenhydrat-haltigen Getränks unmittelbar nach körperlicher Tätigkeit aufgrund einer Kompensations-Wirkung nicht zur Reduktion des RQ kam. Insbesondere ist bekannt, dass der Verzehr von hoch-glykämischen und niedrig-glykämischen Kohlenhydraten unmittelbar nach körperlicher Belastung zu einer höheren Kohlenhydrat-Oxidation und damit einem größeren RQ verglichen zur Oxidation vor Belastungsbeginn führt (Tittelbach et al., 2000). Aus Burke et al., (1998) ist auch bekannt, dass die glykämische Qualität einer ersten Mahlzeit, dargestellt am Vergleich einer Mahlzeit mit einem hohen glykämischen Index mit einer Mahlzeit mit einem niedrigen glykämischen Index, nach Verzehr weiterer Kohlenhydrate zu einem späteren Zeitpunkt, das heisst nach Aufnahme einer zweiten Mahlzeit (sogenanntes "second meal"), keine Bedeutung für den danach erhaltenen, respiratorischen Quotienten hat, da in beiden Fällen ein identischer respiratorischer Quotient erhalten wurde.

Wünschenswert ist es in vielen Fällen jedoch, dass auch nach einer körperlichen Belastung aufgenommene Nahrungsmittel nicht zu einem erhöhten RQ, insbesondere im Vergleich zu dem RQ vor Belastungsbeginn führen, sondern dass vielmehr auch nach Ende der körperlichen Belastung ein besonders niedriger respiratorischer Quotient vorliegt, selbst wenn Kohlenhydrat-haltige Nahrungsmittel nach Belastungsende verzehrt werden. Insbesondere ist es wünschenswert, wenn ein solchermaßen reduzierter, niedriger respiratorischer Quotient nicht nur unmittelbar nach Verzehr einer Mahlzeit, sondern darüber hinaus auch nach der Belastung und nach Verzehr einer weiteren, das heisst einer zweiten Mahlzeit, reduziert bliebe. Im Hinblick auf eine angestrebte verbesserte Regeneration des Körpergewebes, insbesondere auch im Hinblick auf seine Zusammensetzung, die möglichst fettarm und glykogenreich sein sollte, und den angestrebten Trainingseffekt ist es wünschenswert, einen niedrigen respiratorischen Quotienten auch nach Beendigung einer körperlichen Belastung und insbesondere auch bei Verzehr von Nahrungsmitteln nach Beendigung einer körperlichen Belastung bereitzustellen.

Obgleich es für bestimmte Kohlenhydrate, z.B. Trehalose oder Isomaltulose, bekannt ist, dass deren Verzehr während einer körperlichen Betätigung zu einer Erhöhung der Fettoxidation führen kann (WO 2005/013720), ging man jedoch bisher davon aus, dass sich eine derartige, durch Kohlenhydrate verursachte Erhöhung der Fettoxidation, auch aufgrund des vorstehend beschriebenen Kompensationseffekts, nicht bei Verzehr der Kohlenhydrate nach Beendigung einer körperlichen Belastung einstellt, sondern vielmehr eine Erhöhung des RQ zu erwarten ist.

Der vorliegenden Erfindung liegt daher das technische Problem zugrunde, auch Individuen, die sich unmittelbar nach einer körperlichen Belastung befinden, Ernährungsmöglichkeiten aufzuzeigen, die den erwünschten Trainingseffekt und die erwünschte Regeneration, insbesondere ihrer Körperkomposition gewährleisten. Erwünscht ist es, trotz Aufnahme von Kohlenhydraten in der Nachbelastungsphase insbesondere die Fettverbrennung zu optimieren, den Fettanteil in der Körperkomposition zu reduzieren und langfristig wirkende Kohlenhydratspeicher, insbesondere Glykogenreserven aufzubauen. Insbesondere liegt der vorliegenden Erfindung auch das technische Problem zugrunde, eine Lehre aufzuzeigen, mit der sich ein vorteilhafter reduzierter, niedrigerer respiratorischer Quotient nicht nur nach einer ersten Nahrungsaufnahme nach Belastungsende, sondern darüber hinaus auch noch über eine anschließende zweite Mahlzeit hinaus erstreckt, das heisst in reduzierter Form erhalten bleibt.

Die vorliegende Erfindung löst dieses technische Problem durch Bereitstellung der Lehre gemäß der unabhängigen Patentansprüche, insbesondere Isomaltulose oder Gemische von Isomaltulose, insbesondere Gemische von Isomaltulose mit anderen Kohlenhydraten, zur Herstellung von funktionellen Nahrungsmitteln zur Verbesserung der Regeneration von einer körperlichen Belastung ausgesetzten Individuen einzusetzen. In besonders vorteilhafter Weise sind diese funktionellen Nahrungsmittel zum Verzehr nach, vorzugsweise unmittelbar nach, z.B. 0 bis 24, 0 bis 12, 0 bis 4 oder 0 bis 2 Stunden nach, Abschluss der körperlichen Belastung bestimmt und geeignet. Unter dem Begriff "unmittelbar nach, zum Beispiel 0 Stunden" wird verstanden, dass die Nahrungsmittel nicht gleichzeitig mit der körperlichen Belastung, sondern nach körperlicher Belastung aufgenommen werden, insbesondere kurz danach, nämlich zum Beispiel eine beziehungsweise wenige Minuten bis zu zum Beispiel 24 Stunden nach Ende der körperlichen Belastung.

Unter dem Begriff "Regeneration" wird im Zusammenhang mit der vorliegenden Erfindung insbesondere eine Regeneration des Körpergewebes, insbesondere der Zusammensetzung des Körpergewebes, die mehrere Stunden bis Tage anhalten kann, verstanden. Eine Regeneration im Sinne der vorliegenden Erfindung ist ein über das Belastungsende hinaus beziehungsweise nach der Belastung anhaltender Aufbau und eine stoffliche Fixierung längerfristig nutzbarer Kohlenhydrate, also Polysaccharide, insbesondere Glykogen, und der Erhalt eines Fettstoffwechsels auf Basis von Fett, das heisst unter Nutzung von Fett, insbesondere ein Abbau von Fettanteilen im Gewebe. Eine Regeneration im Sinne der vorliegenden Erfindung ist ein Umbau und Aufbau der Fett- und Kohlenhydratzusammensetzung des Körpers, der gleichzeitig mit einer Reduktion des Fettanteils in einem Aufbau längerfristig nutzbarer Kohlenhydrate, insbesondere Glykogen resultiert.

Im Zusammenhang mit der vorliegenden Erfindung wird unter einer verbesserten Regeneration insbesondere auch ein Umbau mit einer Veränderung der Körperkomposition verstanden, die zu einem reduzierten Fett- und einem erhöhten Glykogenanteil in dieser führt. Eine verbesserte Regeneration im Sinne der vorliegenden Erfindung ist daher ein zu einem höheren Glykogen- und einem geringeren Fettanteil in der Körperzusammensetzung führender Stoffwechselvorgang.

Eine Regeneration im Sinne der vorliegenden Erfindung ist streng zu unterscheiden von einer Wiederauffüllung kurzfristig verfügbarer E-nergiereserven (sog. "recovery"), zum Beispiel Glucosemonosaccharide und ATP.

Überraschenderweise wurde gefunden, dass der Verzehr von Isomaltulose und Isomaltulose-haltigen Gemischen nach körperlicher Anstrengung zu einem besonders niedrigen respiratorischen Quotienten führt, der im Wesentlichen gleich groß oder sogar geringer als unmittelbar vor der Belastung ist, was angesichts des vorstehend diskutierten Standes der Technik nicht zu erwarten war. Dagegen führte der Verzehr anderer Kohlenhydrate nach körperlicher Belastung, z.B. von Maltodextrin, zu einem respiratorischen Quotienten nach körperlicher Belastung, der sogar um 13 % höher als vor der Belastung war. Auch dann, wenn Kohlenhydrat-freie Nahrungsmittel verzehrt wurden, das heißt bei einem Placebo, war der respiratorische Quotient nach Belastung um rund 6 % deutlich höher als vorher.

In einer besonders bevorzugten Ausführungsform wurde gefunden, dass der Verzehr von Isomaltulose und Isomaltulose-haltigen Gemischen nach körperlicher Anstrengung zu einem wie vorstehend ausgeführt besonders niedrigen respiratorischen Quotienten führt, der sich überraschenderweise auch noch weit über das Belastungsende hinaus, auch über eine zweite Mahlzeit hinaus, in dieser reduzierten Form erstreckt. Die vorliegende Erfindung stellt daher einen sogenannten "second meal"-Effekt für Isomaltulose bereit. In Zusammenhang mit der vorliegenden Erfindung wird unter einem "second meal"-Effekt verstanden, dass sich der nach Isomaltuloseverzehr und Belastung einstellende respiratorische Quotient viel niedriger als bei einem hoch-glykämischen Nahrungsmittel ist und dass sich dieser niedrige respiratorische Quotient auch noch im Unterschied zu den hoch-glykämischen Nahrungsmitteln nach Verzehr einer zweiten Mahlzeit erhielt, das heisst in dieser niedrigen Form erhalten blieb.

In einer Ausführungsform betrifft die vorliegende Erfindung die Verwendung von Isomaltulose oder Gemischen von Isomaltulose zur Herstellung von funktionellen Nahrungsmitteln zur Bereitstellung eines "second meal"-Effektes, insbesondere in bevorzugter Ausführungsform eines "second meal"-Effekts, der angezeigt wird durch einen nach einer Belastung und nach einer sich anschließenden zweiten Nahrungsaufnahme vorliegenden respiratorischen Quotienten, der im Vergleich zu einem unmittelbar vor einer ersten Nahrungsaufnahme und einer körperlichen Belastung vorliegenden respiratorischen Quotienten desselben Individuums im wesentlichen kleiner oder zumindest gleich groß ist.

Unter einem "second meal"-Effekt wird erfindungsgemäß verstanden, dass ein nach einer ersten Mahlzeit eingestellter RQ im wesentlichen unverändert bleibt auch nach Einnahme einer zweiten Mahlzeit, wobei eine zweite Mahlzeit eine Mahlzeit ist, die zeitlich nach der ersten Mahlzeit eingenommen wird.

In einer besonders bevorzugten Ausführungsform ist die zweite Mahlzeit, ebenso wie die erste Mahlzeit, eine Mahlzeit, die funktionelle Nahrungsmittel der vorliegenden Erfindung, das heisst Isoma-Itulose oder Isomaltulose-haltige Gemische aufweist. In einer weiteren, ebenfalls bevorzugten Ausführungsform ist vorgesehen, dass die zweite Mahlzeit eine Isomaltulose-freie beziehungsweise Isoma-Itulosegemisch-freie Mahlzeit ist.

In einer besonders bevorzugten Ausführungsform ist eine Verwendung vorgesehen, wobei die Nahrungsmittel dafür geeignet und bestimmt sind, in Form einer ersten Mahlzeit von zwei zeitlich versetzten Mahlzeiten nach Abschluss der körperlichen Belastung verzehrt zu werden und wobei die verbesserte Regeneration durch einen nach Belastung und nach Verzehr beider Mahlzeiten vorliegenden respiratorischen Quotienten angezeigt wird, der im Vergleich zu einem unmittelbar vor der körperlichen Belastung vorliegenden respiratorischen Quotienten desselben Individuums im wesentlichen gleich groß oder kleiner ist und wobei die Nahrungsmittel zumindest der ersten Mahlzeit, vorzugsweise beider Mahlzeiten zum Verzehr nach Abschluss der körperlichen Belastung bestimmt und geeignet sind. In einer besonders bevorzugten Ausführungsform ist vorgesehen, dass die zweite Mahlzeit keine Isomaltulose oder Isomaltulose-haltige Gemische enthält, also Isomaltulose-frei ist. Selbstverständlich kann jedoch auch vorgesehen sein, dass die zweite Mahlzeit ebenso wie die erste Mahlzeit Isomaltulose oder Isomaltulose-haltige Gemische enthält und damit ein funktionelles Nahrungsmittel der vorliegenden Erfindung ist.

In einer bevorzugten Ausführungsform sind die Nahrungsmittel dafür geeignet und bestimmt, in Form von mindestens einer, vorzugsweise zwei zeitlich versetzten oder beabstandeten Mahlzeiten, nach Abschluss der körperlichen Belastung verzehrt zu werden.

In einer Ausführungsform betrifft die vorliegende Erfindung die Verwendung von Isomaltulose oder Gemischen davon zur Herstellung von funktionellen Nahrungsmitteln zur Bereitstellung eines "second meal"-Effektes, der angezeigt wird durch einen nach einer zweiten Nahrungsaufnahme vorliegenden respiratorischen Quotienten, der im Vergleich zu einem unmittelbar vor einer ersten Nahrungsaufnahme vorliegenden respiratorischen Quotienten desselben Individuums im wesentlichen kleiner oder zumindest gleich groß ist.

In einer bevorzugten Ausführungsform ist die zweite Nahrungsaufnahme nach der einzigen oder, wenn zwei Belastungen vorgesehen sind, nach der zweiten, zum Beispiel 30-minütig andauernden, körperlichen Belastung vorgesehen.

Die vorliegende Erfindung betrifft in einer besonders bevorzugten Ausführungsform die Verwendung von Isomaltulose oder Gemischen von Isomaltulose zur Herstellung von funktionellen Nahrungsmitteln zur Verbesserung der Regeneration von einer körperlichen Belastung ausgesetzten Individuen, wobei diese verbesserte Regeneration durch einen nach Belastung und anschließender Nahrungsmittelaufnahme vorliegenden respiratorischen Quotienten angezeigt wird, der im Vergleich zu einem unmittelbar vor der körperlichen Belastung vorliegenden respiratorischen Quotienten desselben Individuums, vorzugsweise im Wesentlichen, gleich groß ist. Vorzugsweise sind diese Nahrungsmittel zum Verzehr nach Abschluss der körperlichen Belastung geeignet und bestimmt.

In einer weiteren bevorzugten Ausführungsform betrifft die Erfindung die vorgenannte Verwendung von Isomaltulose oder Gemischen von Isomaltulose zur Herstellung von funktionellen Nahrungsmitteln, wobei die verbesserte Regeneration durch einen nach Belastung und anschließender Nahrungsmittelaufnahme vorliegenden respiratorischen Quotienten angezeigt wird, der im Vergleich zu einem unmittelbar vor der körperlichen Belastung vorliegenden respiratorischen Quotienten desselben Individuums, vorzugsweise im Wesentlichen, kleiner als dieser ist. Vorzugsweise sind diese Nahrungsmittel zum Verzehr nach Abschluss der körperlichen Belastung geeignet und bestimmt.

In einer weiteren bevorzugten Ausführungsform betrifft die vorliegende Erfindung die Verwendung von Isomaltulose oder Gemischen von Isomaltulose zur Herstellung von funktionellen Nahrungsmitteln zur Verbesserung der Regeneration von einer körperlichen Belastung ausgesetzten Individuen, wobei die verbesserte Regeneration durch einen respiratorischen Quotienten angezeigt wird, der bei Verwendung von Isomaltulose beziehungsweise Gemischen von Isomaltulose im Vergleich zu der Verwendung von anderen Kohlenhydraten im ansonsten gleichen Nahrungsmittel, insbesondere im Vergleich zu hoch-glykämischen Kohlenhydraten, z.B. Glucose, Maltodextrin, Glucosesirup oder Saccharose, bei dem selben Individuum geringer ist.

In einer weiteren bevorzugten Ausführungsform betrifft die vorliegende Erfindung die Verwendung von Isomaltulose oder Gemischen von Isomaltulose zur Herstellung von funktionellen Nahrungsmitteln zur Verbesserung der Regeneration von einer körperlichen Belastung ausgesetzten Individuen, wobei die verbesserte Regeneration durch einen respiratorischen Quotienten angezeigt wird, der bei Verwendung von Isomaltulose oder Gemischen von Isomaltulose in dem funktionellen Nahrungsmittel bei dem selben Individuum geringer ist als wenn in den ansonsten gleichen funktionellen Nahrungsmitteln keine Kohlenhydrate eingesetzt werden.

In einer bevorzugten Ausführungsform der vorliegenden Erfindung ist eine Verwendung bereitgestellt, gemäß der die Isomaltulose oder diese enthaltende Gemische auch den Anstieg eines RQ reduziert, der ohne die Anwesenheit der Isomaltulose oder dem Gemisch in einem ansonsten gleichen Nahrungsmittel durch die Anwesenheit von anderen Kohlenhydraten in dem funktionellen Nahrungsmittel verursacht wird. Isomaltulose wirkt in dieser erfindungsgemäßen Verwendung als Modulator oder Einflussgröße auf die durch andere Kohlenhydrate bewirkte Regeneration der Körperkomposition, insbesondere den RQ körperlich belasteter Individuen.

In einer weiteren bevorzugten Ausführungsform betrifft die Erfindung die vorliegenden Verwendungen, wobei der der verbesserten Regeneration entsprechende respiratorische Quotient 0 bis 24, vorzugsweise 0 bis 12, insbesondere 0 bis 4, vorzugsweise 0 bis 2 Stunden nach Abschluss der körperlichen Belastung und dem Verzehr der Isomaltulose-haltigen Nahrungsmittel vorliegt, wobei unter "0 Stunden" insbesondere mindestens eine oder mindestens mehrere Minuten zu verstehen sind. In besonders bevorzugter Ausführungsform wird der erfindungsgemäß erhaltene, der verbesserten Regeneration entsprechende respiratorische Quotient nicht allein kurzfristig, sondern vielmehr kurz- und langfristig, vorzugsweise langfristig, zum Beispiel für einen Zeitraum von 3 bis 24, 4 bis 24, 5 bis 24 oder 6 bis 24 Stunden nach Abschluss der körperlichen Belastung und der danach unmittelbar, das heisst kurz danach erfolgenden Nahrungsaufnahme, erhalten.

Unter dem Begriff "0 Stunden nach Belastung" wird verstanden, dass der respiratorische Quotient unmittelbar nach Abschluss der körperlichen Belastung, das heisst nicht gleichzeitig, wohl aber direkt danach, zum Beispiel eine oder wenige Minuten danach bis zu zum Beispiel 24 Stunden nach Ende der körperlichen Belastung vorliegt.

In einer weiteren bevorzugten Ausführungsform betrifft die vorliegende Erfindung die vorliegenden Verwendungen, wobei der unmittelbar vor der körperlichen Belastung vorliegende respiratorische Quotient von 60 bis 0, vorzugsweise 60 bis 1 Minute, insbesondere 30 bis 0 Minuten, vorzugsweise 30 bis 1 Minute vor Beginn der körperlichen Belastung vorliegt.

In einer weiteren bevorzugten Ausführungsform betrifft die vorliegende Erfindung die vorliegende Verwendung, wobei die körperliche Belastung einem Energieverbrauch von 0,02 bis 0,5 kcal/kg Körpergewicht/Minute entspricht.

Die vorliegende Erfindung betrifft in bevorzugter Weise die vorgenannten Verwendungen, wobei das Individuum ein Mensch oder ein Tier, insbesondere ein Säuger, vorzugsweise ein Mensch ist.

Die vorliegende Erfindung betrifft in einer weiteren bevorzugten Ausführungsform ein Nahrungsmittel, welches in Form eines flüssigen Nahrungsmittels, z.B. einer Ernährungslösung oder eines Getränkes, eines festen Nahrungsmittels oder eines halbfesten Nahrungsmittels vorliegt.

In einer weiteren bevorzugten Ausführungsform ist das Nahrungsmittel ein Softdrink, ein Fruchtsaftgetränk, eine enterale Ernährungslösung, ein hypotonisches Getränk, ein isotonisches Getränk, ein hypertonisches Getränk, ein Energiegetränk, ein Teegetränk, ein Kaffeegetränk, ein Sportgetränk, ein Kakaogetränk, ein Energiegetränk, ein Milchgetränk oder ein Getränkepulver.

In einer weiteren bevorzugten Ausführungsform ist das Nahrungsmittel ein Energieriegel, ein Müsliprodukt, ein Milchprodukt, ein Molkereiprodukt, ein Genussmittel oder eine Backware.

In einer weiteren bevorzugten Ausführungsform beträgt die Konzentration an Isomaltulose im Nahrungsmittel von 1 % bis 99,9 %, vorzugsweise von 1, 2, 3, 4, 5, 6, 7, 8, 9, 10, 20, 30, 40, 50, 60, 70, 80 oder 90 bis 99 Gew.-%, insbesondere von 20 bis 90, 30 bis 80 oder 40 bis 70 Gew.-% (jeweils bezogen auf Trockensubstanz).

Die Isomaltulose kann in einer Ausführungsform auch in Form eines Gemisches vorliegen. Im Zusammenhang mit der vorliegenden Erfindung wird in besonders bevorzugter Ausführungsform unter dem Begriff "Gemische von Isomaltulose" verstanden, dass die Isomaltulose mit geeigneten weiteren Substanzen in Mischungen vorliegen kann, zum Beispiel Stärke, Stärkederivate, Dextrine, zum Beispiel Nutriose, Inulin, Fructooligosaccharide, Leucrose oder Trehalose. In besonders bevorzugter Ausführungsform liegt in einem solchen Gemisch die Isomaltulose in einer Menge von 30 bis 70, vorzugsweise 40 bis 60 Gew.-% und die wenigstens eine Mischungskomponente in einem Mengenverhältnis von 70 bis 30, vorzugsweise 60 bis 40 Gew.-% (jeweils Trockensubstanz bezogen auf das Gemisch) vor. Derartige Gemische können in bevorzugter Ausführung beispielsweise 1 bis 20 Gew.-% Isomaltulose und 80 bis 99 Gew.-% andere Substanzen, z.B. andere Kohlenhydrate oder Intensiv-Süßstoffe enthalten. Derartige Gemische können aber auch 70 bis 99 Gew.-% Isomaltulose und 1 bis 30 Gew.-% andere Substanzen, z.B. Kohlenhydrate oder Intensiv-Süßstoffe enthalten. Selbstverständlich erfasst die Erfindung aber auch andere Gemischverhältnisse von Isomaltulose und anderen Substanzen, z.B. Kohlenhydraten oder Intensiv-Süßstoffe, z.B. von 20 bis 70 Gew.-% Isomaltulose und 30 bis 80 Gew.-% andere Substanzen.

In einer weiteren bevorzugten Ausführungsform betrifft die vorliegende Erfindung ein vorliegendes Gemisch enthaltend Isomaltulose, wobei dieses Gemisch frei ist von Saccharose, frei ist von Glucose, frei ist von Lactose, frei ist von Fructose, frei ist von Sorbit, frei ist von Xylit, frei ist von Mannit oder frei ist von einem oder mehreren oder allen der genannten Zucker oder Zuckeralkohole.

In einer besonders bevorzugten Ausführungsform ist die Isomaltulose das einzig und alleinig in dem funktionellen Nahrungsmittel vorkommende körpergebende süßende Agens. In einer weiteren bevorzugten Ausführungsform betrifft die Erfindung ein vorgenanntes funktionelles Nahrungsmittel, wobei Isomaltulose der einzig und alleinig in dem funktionellen Nahrungsmittel vorkommende Zucker ist.

Im Zusammenhang mit der vorliegenden Erfindung werden unter dem Begriff süßendes Agens Substanzen verstanden, die Süßkraft aufweisen und z.B. Lebensmitteln oder Getränken zugesetzt werden, um einen Süßegeschmack hervorzurufen. Im Zusammenhang mit der vorliegenden Erfindung werden die süßenden Agenzien unterteilt in Zucker, wie Isomaltulose, Saccharose, Glucose oder Fructose, die Körper und Süßkraft geben, sowie Süßungsmittel, also Stoffe, die keine Zucker sind, aber trotzdem Süßkraft aufweisen, und wobei diese wiederum untergliedert werden in Zuckeraustauschstoffe, also süßende Agenzien, die einen Körper und einen physiologischen Brennwert zusätzlich zu einer Süßkraft aufweisen, und Intensivsüßstoffe, also Stoffe, die in der Regel eine sehr hohe Süßkraft, aber keinen Körper und in der Regel keinen oder nur einen geringfügigen physiologischen Brennwert aufweisen.

In einer weiteren bevorzugten Ausführungsform ist das vorgenannte funktionelle Nahrungsmittel für die spezielle Ernährung von Sportlern, Übergewichtigen, Fettleibigen, Diabetikern oder Älteren geeignet.

In einer weiteren bevorzugten Ausführungsform sieht die Erfindung die - nicht beanspruchte - Verwendung von Isomaltulose oder Gemischen von Isomaltulose zur Herstellung von funktionellen Nahrungsmitteln zur Verbesserung der Wirkung körperlicher Belastung auf Individuen vor.

In einer weiteren bevorzugten Ausführungsform betrifft die vorliegende Erfindung die - nicht beanspruchte - Verwendung von Isoma-Itulose oder Gemischen von Isomaltulose zur Herstellung von funktionellen Nahrungsmitteln zur Erzielung eines körperlichen Trainingseffektes auf Individuen.

Die Erfindung betrifft neben den vorgenannten Verwendungsaspekten selbstverständlich auch die entsprechenden Verfahrenslehren, nämlich - nicht beanspruchte - Verfahren zur Verbesserung der Regeneration von einer körperlichen Belastung ausgesetzten Individuen, wobei diesen Individuen in Form von funktionellen Nahrungsmitteln Isomaltulose oder Gemische von Isomaltulose zugeführt werden, insbesondere nach Abschluss der körperlichen Belastung, vorzugsweise unmittelbar mit Abschluss. Weitere vorteilhafte Verfahrensaspekte ergeben sich aus den vorgenannten Verwendungsaspekten.

Weitere vorteilhafte Ausgestaltungen ergeben sich aus den Unteransprüchen.

Die Erfindung wird anhand der folgenden Beispiele und der dazugehörigen Figuren näher erläutert.

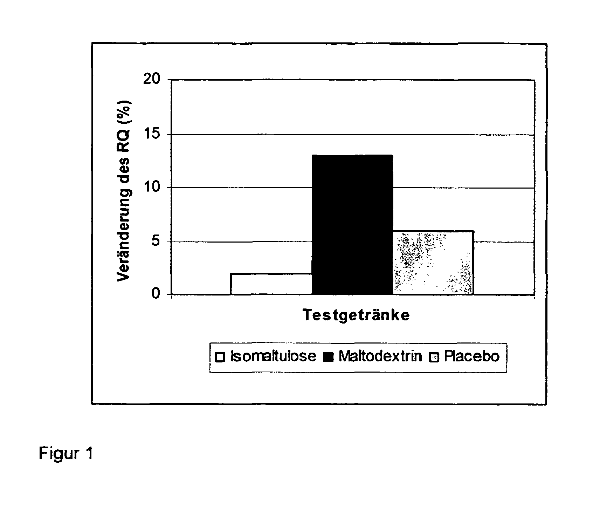

Die Figur 1 zeigt die prozentuale Veränderung des respiratorischen Quotienten (RQ) nach Verzehr von Getränken ohne Kohlenhydrate, mit Isomaltulose und mit Maltodextrin.

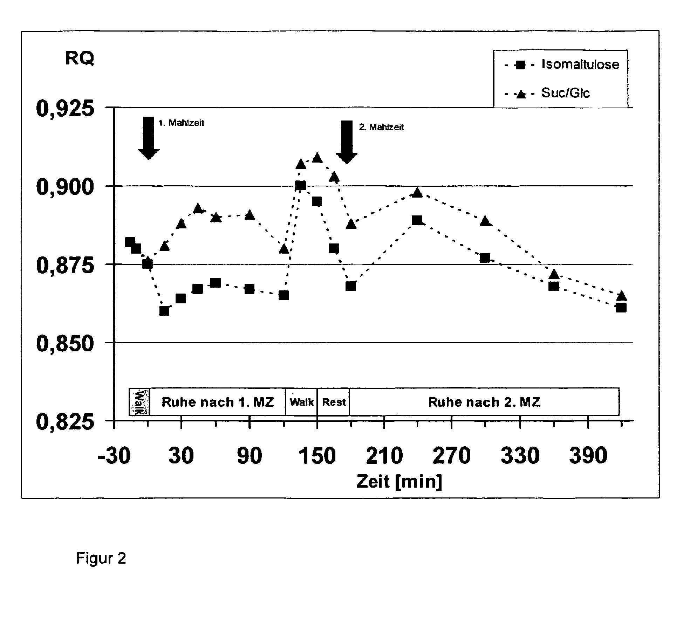

Die Figur 2 zeigt den Verlauf des RQ über eine Testperiode mit zwei Mahlzeiten.

### Literatur

1. Dionne et al., Am J Clin Nutr (1999) 69, 927-30.
2. Saris et al., Int J Obes Relat Metab Disoerd (2004) 28(6), 759-65.
3. Tittelbach et al., Obes Res (2000) 8, 496-505.
4. Achten & Jeukendrup, J Sports Sci (2003) 21, 1017-1024
5. Schneiter et al., Am J Physiol (1995) 269, E1031-6
6. Brand-Miller et al., Am J Clin Nutr (2002) 76, 281-5
7. Burke et al., J. Appl. Physiol. (1998) 85, 2220-2226

### Beispiel 1

Die Wirkung von Isomaltulose (Palatinose™) auf die Stoffwechselregulation vor, während und nach körperlicher Belastung wurde untersucht und mit dem Verzehr von Maltodextrin oder einem nichtglykämisch wirkenden Placebo mit äquivalenter Süße verglichen.

**Isomaltulose - Formulierung**

| Pos. | Rohstoff | Menge % |
|---|---|---|
| 1 | Isomaltulose | 93,98 |
| 2 | Citronensäure (wasserfrei) | 2,50 |
| 3 | Farbstoff 311744 Eurocert Quinoline Yellow | 0,004 |
| 4 | Citronenaroma 210336 Fa. Symrise | 1,00 |
| 5 | Sucralose | 0,03 |
| Summe | | 100,00 |

**Maltodextrin - Formulierung**

| Pos. | Rohstoff | Menge % |
|---|---|---|
| 1 | Maltodextrin, 2022225 Fa. Agrana | 93,96 |
| 2 | Citronensäure (wasserfrei) | 2,50 |
| 3 | Farbstoff 311744 Eurocert Quinoline Yellow | 0,004 |
| 4 | Citronenaroma 210336 Fa. Symrise | 1,00 |
| 5 | Sucralose | 0,05 |
| Summe | | 100,00 |

**Placebo - Formulierung (Inulin-Basis)**

| Pos. | Rohstoff | Menge % |
|---|---|---|
| 1 | Inulin RAFTILINE ST-Gel (Instant) | 64,46 |
| 2 | Citronensäure (wasserfrei) | 25,00 |
| 3 | Farbstoff 311744 Eurocert Quinoline Yellow | 0,04 |
| 4 | Citronenaroma 210336 Fa. Symrise | 10,00 |
| 5 | Sucralose | 0,50 |
| Summe | | |

Die Untersuchung wurde als 3-armige Cross-over-Studie an 21 Probanden durchgeführt. Bei jedem Probanden wurde die Wirkung eines nicht-glykämischen Getränks (Placebo = gesüßtes Mineralwasser) versus eines Getränks mit Maltodextrin versus Isomaltulose getestet.

Die Probanden waren männliche Ausdauersportler mit mehrjähriger Trainingserfahrung (VO2max > 55 ml/kg KG) im Alter zwischen 23 und 50 Jahren.

In einer Stufenergometrie auf dem Rad (100 Watt Beginn; Steigerung um 50 Watt/3min.) wurde zunächst die Leistung im Bereich von 70-75 % der maximalen Sauerstoffaufnahme bestimmt. Nach einer ausreichenden Regenerationsphase von mindestens 5 Tagen erfolgte die erste Testung in randomisierter Reihenfolge. Die Testung fand morgens, jeweils zur gleichen Uhrzeit, 3-4 Stunden nach einem standardisierten Frühstück statt.

Es erfolgte zunächst eine standardisierte Ausdauerbelastung auf dem Fahrradergometer bei 70-75 % der VO2max über 90 min. An die 90-minütige submaximale Belastung wurde unmittelbar ein anaerober Wingate-Test angeschlossen. 250 ml des jeweiligen Getränks mit 25 g Isomaltulose bzw. Maltodextrin bzw. ohne KH wurden 30 min vor der Belastung, unmittelbar zu Belastungsbeginn, nach 45 min. der Ausdauerbelastung sowie unmittelbar nach Beendigung des Wingate-Tests (hier jetzt als 2x 250 ml) zugeführt. Eine Bestimmung des respiratorischen Quotienten (RQ) erfolgte zu den Zeitpunkten -30, -15, 0, 15, 30, 45, 60 75, 90 min vor beziehungsweise während der Belastung, unmittelbar nach Abbruch des Wingate-Tests sowie 3, 15, 30, 60 und 120 min nach Belastungsende. Weitere getestete Parameter waren Glukose, Laktat, freie Fettsäuren, Insulin.

Die statistische Auswertung erfolgte mit SPSS Version 13.1. Die Daten wurden auf signifikante Unterschiede (in Abhängigkeit des zugeführten Getränkes) mittels des Wilcoxon-Tests für gepaarte Daten geprüft. Ein p-Wert < 0.05 wurde als signifikant erachtet. Die Mehrfachtestung wurde über eine Korrektur nach Holm berücksichtigt.

Während der gesamten Testphase zeigt sich nach Isomaltulose-Zufuhr ein niedrigerer RQ. Der RQ war überraschenderweise nach der körperlichen Belastung beim Isomaltulose-Getränk im Wesentlichen gleich groß wie unmittelbar vor der Belastung. Hingegen war der respiratorische Quotient nach der körperlichen Belastung beim Maltodextrin-Getränk um 13% höher und beim Placebo um rund 6% höher als unmittelbar vor der Belastung. Dies zeigt auch die Figur, in der die prozentualen Veränderungen der RQ-Werte nach gegenüber vor der körperlichen Belastung dargestellt sind.

### Beispiel 2: Rezeptur für ein Orangen-Getränk

**Isomaltulose-Instant-Getränk Orange mit L-Carnitin**

| Pos. | Zutaten | Anteil |
|---|---|---|
| 1 | Isomaltulose | 92,60 % |
| 2 | Zitronensäure (wasserfrei) | 4,96 % |
| 3 | Tri-Natrium-Citrat | 0,26 % |
| 4 | Tri-Calcium-Phosphat | 0,22 % |
| 5 | Vitamin C | 0,24 % |
| 6 | Trübungsmittel mit Farbstoff E 171 | 0,48 % |
| 7 | Farbstoff E 102 (85 %) | 0,01 % |
| 8 | Farbstoff E 110 (85 %) | 0,016 % |
| 9 | Gummi Arabicum (sprühgetrocknet) E 414 | 0,10 % |
| 10 | Xanthan E 415 | 0,10 % |
| 11 | Na-Carboxymethylcellulose E 466 | 0,10 % |
| 12 | Orangen-Aroma Typ 100 | 0,64 % |
| 13 | Orangen-Aroma Typ 120 | 0,24 % |
| 14 | Sucralose | 0,03 % |
| Summe | | 100,0 % |

### Beispiel 3: Rezeptur für ein Sportlergetränk

**Isomaltulose-Sport-Drink mit L-Carnitin**

| Pos. | Zutaten | Anteil |
|---|---|---|
| 1 | Isomaltulose | 90,04 % |
| 2 | Zitronensäure (wasserfrei) | 6,360 % |
| 3 | Vitamin C | 0,550 % |
| 4 | Tri-Natrium-Citrat | 1,194 % |
| 5 | Trübungsmittel mit Farbstoff E 171 | 0,262 % |
| 6 | Xanthan E 415 | 0,091 % |
| 7 | Na-Carboxymethylcellulose E 466 | 0,091 % |
| 8 | Sucralose | 0,300 % |
| 9 | Farbstoff E 102 (85 %) | 0,018 % |
| 10 | Aroma Grapefruit-Lemon | 1,090 % |
| Summe | | 100,00 % |

### Beispiel 4: Rezeptur für ein ACE-Getränk

**Isomaltulose-ACE-Drink**

| Pos. | Zutaten | Anteil |
|---|---|---|
| 1 | Isomaltulose | 94,52 % |
| 2 | Zitronensäure (wasserfrei) | 3,88 % |
| 3 | Tri-Natrium-Citrat | 0,27 % |
| 4 | Tri-Calcium-Phosphat | 0,25 % |
| 5 | Trübungsmittel mit Farbstoff E 171 | 0,30 % |
| 7 | Farbstoff E 110 (85 %) | 0,033 % |
| 8 | Farbstoff E 102 (85 %) | 0,0125% |
| 9 | Farbstoff Coffeebrown TF 8 | 0,0025% |
| 10 | Farbstoff E 129 (Allura Red) | 0,0015% |
| 11 | Vitamin E | 0,02 % |
| 12 | Provitamin A | 0,032 % |
| 13 | Xanthan E 415 | 0,170 % |
| 14 | Na-Carboxymethylcellulose E 466 | 0,170 % |
| 15 | Multifrucht-Aroma | 0,330 % |
| Summe | | 100,00% |

### Beispiel 5: "Second Meal"-Effekt

Die Untersuchung war eine Cross-over-Studie mit 20 Probanden. Bei jedem Probanden wurde die Wirkung von Isomaltulose vs. Saccharose/Glukosesirup getestet.

Das Testintervall umfasste zwei Mahlzeiten, in die die Prüfsubstanzen integriert waren, zum einen ein Frühstück, das aus einem Getränk (250ml, 10% KH = 25g Prüfsubstanz) und Keksen (ca. 140g, ebenfalls mit 25g Prüfsubstanz) bestand und zum anderen ein Mittagessen, bestehend aus Mini-Pizzas, einem Apfel und einem Erfrischungsgetränk. Die pro Portion Mürbekekse verzehrte KH-Menge aus Weizenstärke des Mehls war bei allen Keks-Varianten mit rund 50-60g KH gleich hoch. Jede Testperiode umfasste zwei postprandiale Phasen.

Zunächst erfolgte die Bestimmung des respiratorischen Quotienten in Ruhe, dann während einer sich anschließenden 30 min Belastung bei moderater Intensität (Laufband Steigeprotokoll 4 km/h 5% Steigung), nach Ablauf der 30-minütigen Nachbelastungsphase sowie bis 4 Stunden nach Verzehr der zweiten Mahlzeit (dem Mittagessen).

Der RQ nach Verzehr des Isomaltulose-Frühstücks fiel ab und war niedriger als nach dem Saccharose/Glukosesirup-Frühstück, während gleichzeitig das Ausmaß, um das der RQ niedriger war, auch nach Verzehr einer zweiten Mahlzeit (Mittagessen) erhalten blieb. Die RQ-Kurven verliefen unmittelbar nach der initialen Reduktion des RQ durch das Isomaltulose-Frühstück parallel bis zum Ende der Testperiode. Der RQ-Ausgangspunkt ("set-point") ist maßgeblich für den RQ über den ganzen Tag.

## Patentansprüche

1. Verwendung von Isomaltulose oder Gemischen von Isomaltulose zur Herstellung von funktionellen Nahrungsmitteln zur Verbesserung der Regeneration von einer körperlichen Belastung ausgesetzten Individuen, wobei die verbesserte Regeneration durch einen nach Belastung vorliegenden respiratorischen Quotienten angezeigt wird, der im Vergleich zu einem unmittelbar vor der körperlichen Belastung vorliegenden respiratorischen Quotienten desselben Individuums im Wesentlichen gleich groß oder kleiner ist und wobei diese Nahrungsmittel zum Verzehr nach Abschluss der körperlichen Belastung bestimmt und geeignet sind.

2. Verwendung nach Anspruch 1, wobei die verbesserte Regeneration durch einen respiratorischen Quotienten angezeigt wird, der bei Verwendung von Isomaltulose oder Gemischen von Isomaltulose im Vergleich zu den Verwendungen von anderen Kohlenhydraten in ansonsten gleichen Nahrungsmitteln bei demselben Individuum geringer ist.

3. Verwendung nach Anspruch 2, wobei die anderen Kohlenhydrate hoch-glykämische Kohlenhydrate sind.

4. Verwendung nach Anspruch 1, wobei die verbesserte Regeneration durch einen respiratorischen Quotienten angezeigt wird, der bei Verwendung von Isomaltulose oder Gemischen von Isomaltulose geringer ist als ohne Verwendung von Kohlenhydraten im ansonsten gleichen Nahrungsmittel bei demselben Individuum.

5. Verwendung nach einem der vorhergehenden Ansprüche, wobei der der verbesserten Regeneration entsprechende respiratorische Quotient von 0 bis 24 Stunden, insbesondere von 0 bis 4 Stunden, nach der körperlichen Belastung vorliegt.

6. Verwendung nach einem der vorhergehenden Ansprüche, wobei der unmittelbar vor der körperlichen Belastung vorliegende respiratorische Quotient von 60 bis 0 Minuten vor der körperlichen Belastung vorliegt.

7. Verwendung nach einem der vorhergehenden Ansprüche, wobei die körperliche Belastung einem Energieverbrauch von 0,02 bis 0,5 kcal/kg Körpergewicht/min entspricht.

8. Verwendung nach einem der vorhergehenden Ansprüche, wobei das Individuum ein Mensch ist.

9. Verwendung nach einem der vorhergehenden Ansprüche, wobei das Nahrungsmittel ein Getränk, ein halbfestes oder ein festes Nahrungsmittel ist.

10. Verwendung nach einem der vorhergehenden Ansprüche, wobei das Nahrungsmittel ein Softdrink, ein Fruchtsaftgetränk, eine enterale Ernährungslösung, ein hypotonisches Getränk, ein isotonisches Getränk, hypertonisches Getränk, ein Energiegetränk, ein Teegetränk, ein Kaffeegetränk, ein Sportgetränk, ein Kakaogetränk, ein Energiegetränk, Milchgetränk oder Getränkepulver ist.

11. Verwendung nach einem der vorhergehenden Ansprüche, wobei das Nahrungsmittel ein Energieriegel, ein Müsliprodukt, ein Milchprodukt, ein Molkereiprodukt, ein Genussmittel oder eine Backware ist.

12. Verwendung nach einem der vorhergehenden Ansprüche, wobei die Konzentration an Isomaltulose am Nahrungsmittel von 1 % bis 99 Gew.-% (bezogen auf Trockensubstanz) beträgt.

13. Verwendung nach einem der vorhergehenden Ansprüche, wobei die Nahrungsmittel dafür geeignet und bestimmt sind, in Form einer ersten Mahlzeit von zwei zeitlich versetzten Mahlzeiten nach Abschluss der körperlichen Belastung verzehrt zu werden und wobei die verbesserte Regeneration durch einen nach Belastung und Verzehr beider Mahlzeiten vorliegenden respiratorischen Quotienten angezeigt wird, der im Vergleich zu einem unmittelbar vor der körperlichen Belastung vorliegendem respiratorischen Quotienten desselben Individuums im Wesentlichen gleich groß oder kleiner ist und wobei zumindest das erste Nahrungsmittel zum Verzehr nach Abschluss der körperlichen Belastung bestimmt und geeignet ist.

14. Verwendung nach Anspruch 13, wobei die zweite Mahlzeit Isomaltulose-haltig ist.

15. Verwendung nach Anspruch 13, wobei die zweite Mahlzeit Isomaltulose-frei ist.

16. Verwendung nach einem der vorhergehenden Ansprüche, wobei das Nahrungsmittel für die spezielle Ernährung von Sportlern, Übergewichtigen, Fettleibigen, Diabetikern oder Älteren geeignet ist.

17. Verwendung von Isomaltulose oder Gemischen von Isomaltulose zur Herstellung von funktionellen Nahrungsmitteln zur Erzielung eines "second meal"-Effektes, **gekennzeichnet durch** einen nach einer Belastung und nach einer sich anschließenden zweiten Nahrungsaufnahme vorliegenden respiratorischen Quotienten, der im Vergleich zu einem unmittelbar vor einer ersten Nahrungsaufnahme und einer körperlichen Belastung vorliegenden respiratorischen Quotienten desselben Individuums im wesentlichen kleiner oder zumindest gleich groß ist.

18. Verwendung von Isomaltulose oder Gemischen von Isomaltulose nach einem der Ansprüche 1 bis 16, wobei die Gemische neben Isomaltulose Kohlenhydrate und/oder Intensiv-Süßstoffe enthalten.

## Claims

1. Use of isomaltulose or mixtures of isomaltulose for the production of functional food products for improving the regeneration of individuals exposed to physical exertion, wherein the improved regeneration is indicated by a respiratory quotient which is present after exertion and which is substantially equal to or lower than a respiratory quotient which is present immediately prior to the physical exertion of the same individual and wherein these food products are intended and suitable for consumption after completion of the physical exertion.

2. Use according to claim 1, wherein the improved regeneration is indicated by a respiratory quotient which, upon use of isomaltulose or mixtures of isomaltulose, is lower compared to the uses of other carbohydrates in otherwise identical food products in the same individual.

3. Use according to claim 2, wherein the other carbohydrates are highglycaemic carbohydrates.

4. Use according to claim 1, wherein the improved regeneration is indicated by a respiratory quotient which, upon use of isomaltulose or mixtures of isomaltulose, is lower than without the use of carbohydrates in the otherwise identical food product in the same individual.

5. Use according to one of the preceding claims, wherein the respiratory quotient corresponding to the improved regeneration is present at from 0 to 24 hours, in particular at from 0 to 4 hours, after the physical exertion.

6. Use according to one of the preceding claims, wherein the respiratory quotient which is present immediately prior to the physical exertion is present at from 60 to 0 minutes prior to the physical exertion.

7. Use according to one of the preceding claims, wherein the physical exertion corresponds to a consumption of energy of from 0.02 to 0.5 kcal/kg of body weight/minute.

8. Use according to one of the preceding claims, wherein the individual is a human being.

9. Use according to one of the preceding claims, wherein the food product is a drink, a semi-solid food product or a solid food product.

10. Use according to one of the preceding claims, wherein the food product is a soft drink, a fruit juice drink, an enteral nutrition solution, a hypotonic drink, an isotonic drink, a hypertonic drink, an energy drink, a tea drink, a coffee drink, a sports drink, a cocoa drink, an energy drink, a milk drink or a drink powder.

11. Use according to one of the preceding claims, wherein the food product is an energy bar, a muesli product, a milk product, a dairy product, a luxury food or a bakery product.

12. Use according to one of the preceding claims, wherein the food product contains a concentration of isomaltulose of from 1% to 99% by weight (based on dry substance).

13. Use according to one of the preceding claims, wherein the food products are suitable and intended to be consumed in the form of a first meal of two meals at different times after completion of the physical exertion and wherein the improved regeneration is indicated by a respiratory quotient which is present after exertion and consumption of both meals and which is substantially equal to or lower than a respiratory quotient which is present immediately prior to the physical exertion of the same individual, and wherein at least the first food product is intended and suitable for consumption after completion of the physical exertion.

14. Use according to claim 13, wherein the second meal contains isomaltulose.

15. Use according to claim 13, wherein the second meal is free of isomaltulose.

16. Use according to one of the preceding claims, wherein the food product is suitable for specific nutrition of sportsmen, overweight persons, obese persons, diabetics or elderly persons.

17. Use of isomaltulose or mixtures of isomaltulose for the production of functional food products for achieving a second meal effect, **characterized by** a respiratory quotient which is present after exertion and a subsequent second ingestion and which is substantially lower than or at least equal to a respiratory quotient which is present immediately prior to a first ingestion and physical exertion of the same individual.

18. Use of isomaltulose or mixtures of isomaltulose according to any one of claims 1 to 16, wherein the mixtures contain, in addition to isomaltulose, carbohydrates and/or intensive sweeteners.

## Revendications

1. Utilisation de l'isomaltulose ou des mélanges de l'isomaltulose pour la fabrication des produits alimentaires fonctionnels pour améliorer la régénération des individus soumis à un effort physique, dans laquelle la régénération améliorée est montrée par un quotient respiratoire obtenu après l'effort physique qui est sensiblement égal ou inférieur par rapport à un quotient respiratoire du même individu obtenu immédiatement avant l'effort physique, et dans laquelle ces produits alimentaires sont destinés et aptes pour la consommation après l'achèvement de l'effort physique.

2. Utilisation selon la revendication 1, dans laquelle la régénération améliorée est montrée par un quotient respiratoire qui est, lors de l'utilisation de l'isomaltulose ou des mélanges de l'isomaltulose, inférieur par rapport à les utilisations des autres glucides dans des produits alimentaires autrement identiques dans le même individu.

3. Utilisation selon la revendication 2, dans laquelle les autres glucides sont des glucides hautement glycémiques.

4. Utilisation selon la revendication 1, dans laquelle la régénération améliorée est montrée par un quotient respiratoire qui est, lors de l'utilisation de l'isomaltulose ou des mélanges de l'isomaltulose, inférieur par rapport au quotient obtenu sans utilisation de glucides dans le produit alimentaire autrement identique dans le même individu.

5. Utilisation selon l'une quelconque des revendications précédentes, dans laquelle le quotient respiratoire correspondant à la régénération améliorée est obtenu de 0 à 24 heures, notamment de 0 à 4 heures, après l'effort physique.

6. Utilisation selon l'une quelconque des revendications précédentes, dans laquelle le quotient respiratoire obtenu immédiatement avant l'effort physique est obtenu de 60 à 0 minutes avant l'effort physique.

7. Utilisation selon l'une quelconque des revendications précédentes, dans laquelle l'effort physique correspond à une consommation d'énergie de 0,02 à 0,5 kcal/kg poids du corps/min.

8. Utilisation selon l'une quelconque des revendications précédentes, dans laquelle l'individu est un homme.

9. Utilisation selon l'une quelconque des revendications précédentes, dans laquelle le produit alimentaire est une boisson, un produit alimentaire semi-solide ou un produit alimentaire solide.

10. Utilisation selon l'une quelconque des revendications précédentes, dans laquelle le produit alimentaire est un soda, une boisson à base de jus de fruits, une solution de nutrition entérale, une boisson hypotonique, une boisson isotonique, une boisson hypertonique, une boisson énergétique, une boisson à base de thé, un boisson à base de café, une boisson pour sportifs, une boisson à base de cacao, une boisson énergétique, une boisson à base de lait ou un poudre de boisson.

11. Utilisation selon l'une quelconque des revendications précédentes, dans laquelle le produit alimentaire est une barre énergétique, un produit de muesli, un produit de lait, un produit laitier, un produit d'agrément ou un produit de boulangerie.

12. Utilisation selon l'une quelconque des revendications précédentes, dans laquelle la concentration de l'isomaltulose dans le produit alimentaire est de 1 % à 99 % en poids (par rapport à la matière sèche).

13. Utilisation selon l'une quelconque des revendications précédentes, dans laquelle les produits alimentaires sont aptes et destinés à être consommés, sous forme d'un premier repas de deux repas décalés dans le temps, après l'achèvement de l'effort physique, et dans laquelle la régénération améliorée est montrée par un quotient respiratoire obtenu après l'effort physique et la consommation des deux repas qui est sensiblement aussi haut ou inférieur par rapport à un quotient respiratoire du même individu obtenu immédiatement avant l'effort physique, et dans laquelle au moins le premier produit alimentaire est destiné et apte à être consommé après l'achèvement de l'effort physique.

14. Utilisation selon la revendication 13, dans laquelle le second repas contient de l'isomaltulose.

15. Utilisation selon la revendication 13, dans laquelle le second repas est exempt de l'isomaltulose.

16. Utilisation selon l'une quelconque des revendications précédentes, dans laquelle le produit alimentaire est apte pour l'alimentation spéciale des sportifs, des personnes souffrant de surpoids, des obèses, des diabétiques ou des personnes âgées.

17. Utilisation de l'isomaltulose ou des mélanges de l'isomaltulose pour la fabrication des produits alimentaires fonctionnels, pour obtenir un effet "second meal", **caractérisée par** un quotient respiratoire obtenu après un effort physique suivi d'un second repas qui est sensiblement inférieur, ou au moins égal, par rapport à un quotient respiratoire du même individu obtenu immédiatement avant un premier repas et un l'effort physique.

18. Utilisation de l'isomaltulose ou des mélanges de l'isomaltulose selon l'une quelconque des revendications 1 à 16, dans laquelle les mélanges contiennent, outre l'isomaltulose, des glucides et/ou des édulcorants intenses.
